(19) Europäisches Patentamt
European Patent Office
Office européen des brevets

(11) Publication number: 0 099 622
B1

(12) **EUROPEAN PATENT SPECIFICATION**

(45) Date of publication of patent specification: **04.12.85**

(51) Int. Cl.$^4$: **C 07 C 85/12,** C 07 C 87/28

(21) Application number: **83302562.0**

(22) Date of filing: **06.05.83**

(54) **Process for the hydrogenation of perhalogenated terephthalonitriles to amines, 2,3,5,6-tetrafluoroxylylene diamine and salts thereof.**

(30) Priority: **27.05.82 GB 8215532**

(43) Date of publication of application:
**01.02.84 Bulletin 84/05**

(45) Publication of the grant of the patent:
**04.12.85 Bulletin 85/49**

(84) Designated Contracting States:
**CH DE FR GB IT LI NL**

(56) References cited:
**GB-A- 970 024**
**US-A-4 320 032**

**CESARE FERRI "Reaktionen der organischen Synthese", 1978, GEORG THIEME VERLAG, Stuttgart, page 92, section 11**

(73) Proprietor: **IMPERIAL CHEMICAL INDUSTRIES PLC**
**Imperial Chemical House Millbank**
**London SW1P 3JF (GB)**

(72) Inventor: **Richardson, Peter John**
**4 Highlands Road**
**Bamford Rochdale, Lancs OL11 5PD (GB)**

(74) Representative: **Houghton, Malcolm John et al**
**Imperial Chemical Industries PLC Legal Department: Patents PO Box 6**
**Welwyn Garden City Herts, AL7 1HD (GB)**

**EP 0 099 622 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a process for hydrogenating perhalogenated terephthalonitriles and to novel compounds which can be obtained therefrom.

Processes for the hydrogenation of terephthalonitrile have been numerously described and as seen from, for example, UK Patent Specifications Nos. 810530, 852972 and 1149251, the hydrogenation is normally carried out under ammoniacal conditions.

Such conditions are not altogether suitable, however, for the hydrogenation of certain halo-substituted terephthalonitriles. For instance, in the case of tetrafluoroterephthalonitrile a high molecular weight material is produced thought to be the result of nucleophilic substitution of ring fluorine atoms by an amine group of the bifunctional hydrogenation product.

According to the present invention there is provided a process for hydrogenating a perhalogenated terephthalonitrile of the formula (I):

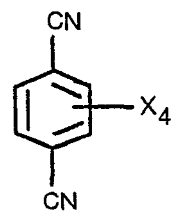

(I)

in which each X is independently fluoro or chloro, to its corresponding amine of formula (II):

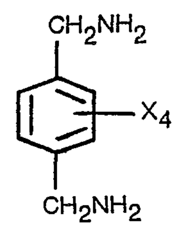

(II)

which comprises reacting under acid conditions the terephthalonitrile with hydrogen in the presence of (i) a hydrogenation catalyst which includes as one component a metal selected from rhodium, palladium, ruthenium, nickel, cobalt and platinum, and (ii) a solvent which is inert to the reaction ingredients and which does not poison the catalyst.

The diamines obtained by this process are useful intermediates in the preparation of pesticidal compounds. 2,3,5,6-Tetrafluoroxylylene diamine and salts thereof are novel compounds and form another aspect of the present invention.

The metal component of the hydrogenation catalyst will usually be present upon a support such as carbon, alumina, alumina-silica, silica, kiesulguhr, calcium carbonate, barium sulfate or bentonite. The metal will usually be present in a proportion of from about 0.1 to 70% by weight, and, in the case of noble metals, generally 1 to 20%. A preferred catalyst is palladium preferably supported on charcoal and especially 5% palladium on charcoal. Nickel and cobalt catalysts, which tend to dissolve in acid conditions, may be less suitable.

The proportions of catalyst to nitrile may be wide-ranging. However, an amount of 0.5 to 5% by weight on nitrile has been found adequate for a catalyst containing a Group 8 noble metal.

The acid used to create the acid conditions is suitably an inorganic acid, particularly an oxyacid and ideally sulphuric acid, although other strong acids, such as hydrochloric acid, may also be suitable. At least an amount of acid chemically equivalent to the amine formed, should be used and preferably an excess up to, for instance, five times the chemically equivalent amount.

It may be prudent to add the acid continuously or intermittently during the process in case too high an acid concentration at the start of reaction, particularly when working at high nitrile concentrations, should have a deleterious effect on product yield.

It is expedient to slurry the barely soluble nitrile, together with all or part of the acid, in the solvent, preferably with water added. The solvent should be one that is inert to the reaction ingredients and does not poison the catalyst. Its choice will be influenced by operational considerations, the solubility in it of the reactant and intermediate and final products and obviously its effect on yield. Particularly suitable solvents are alcohols, especially aliphatic monohydric alcohols of the formula ROH, in which R is $C_{1-6}$ alkyl, and more especially methanol and ethanol. The presence of water gives processing advantages with respect to yield and an ability to operate at lower temperatures and pressures.

For ease of operation, it is desirable to have sufficient solvent and water, if used, present to provide a stirrable nitrile slurry. Suitably, the concentration of nitrile in the total reaction mixture will be from 3 to 15% by weight and even up to 25% by weight, particularly if the acid is added continuously or

intermittently during the process. The proportion of water to solvent is usefully in the range of from 1:50 to 1:1 parts by weight.

The pressure and temperature of hydrogenation may vary within wide limits and will be chosen to suit the hydrogenation equipment available and to avoid too slow a reaction. Suitably pressures of from 1 to 100 or more atmospheres, conveniently from 1 to 30 atmospheres may be used at temperatures ranging from, for example, 0°C to 200°C and typically from 10°C to 120°C. Care must be exercised in working at higher temperatures to avoid dehalogenation of the product. For instance, prolonged processing at 150°C can lead to defluorination of tetrafluoroxylylene diamine.

In carrying out the process of the invention, the nitrile starting material is conveniently charged to a glass lined or stainless steel rotary or stirred autoclave and slurried with all or part of the acid in the solvent and water, if used. The autoclave is pressurised to the desired extent with hydrogen and rotated or stirred at the desired temperature until sufficient hydrogen has been absorbed.

The amine hydrogenation product is obtained as a salt which, in the solid phase, may be recrystallised from a water/solvent mixture and isolated by conventional techniques. The amine may be obtained in its pure form by solvent extraction techniques from a strongly alkaline solution of its salt.

Tetrachloroterephthalonitrile may be obtained from commercially available tetrachloroterephthaloyl chloride by treatment of the latter with aqueous ammonia to form the diamide which can then be dehydrated with, for example, phosphorus oxychloride. Other starting materials may be obtained by replacement with fluoro of one or more of the chloro substituents of the tetrachloroterephthalonitrile. Thus, tetrafluoroterephthalonitrile may be obtained by fluorinating the corresponding tetrachlorinated compound with potassium fluoride in a polar aprotic solvent.

The invention is illustrated by the following Examples 1 to 30 in which percentages are by weight. Percentage yields of diamine are molar; yields by byproducts are computed from GLC traces assuming the same molar response factors as for the diamine. In all Examples, save Example 7 (q.v.), conversion of the dinitrile starting material was 100%. Example 31 is included for comparative purposes only.

## Example 1

Tetrafluoroterephthalonitrile (5.0 g), methanol (70 ml), water (2 ml), 98% sulphuric acid (3 g) and 5% palladium on carbon catalyst (0.25 g) were loaded into the glass liner of rotating autoclave, purged with nitrogen, and then pressurised with hydrogen to 15 atmospheres. The autoclave was rotated for 6 hours at 75°C. The resulting slurry was filtered and the residue slurried with water. The aqueous solution was filtered to remove catalyst, then the water removed by heat until a crust formed on the liquid surface. 74 OP Ethanol was added to give 5.06 g of a white solid precipitate. This was dissolved in 5N sodium hydroxide and extracted repeatedly with ether. The residue after evaporation of the ether was recrystallised from toluene to give pure 2,3,5,6-tetrafluoroxylylene diamine (m.pt.89°C).

I.R. (KBr 3385, 3275, 2955, 1600, 1480, 1348, 1268, 1165, 987, 928, 878, 828. 700 $cm^{-1}$.

Proton nmr 2.096 and 3.78δ, consistent with (2p, s, —$NH_2$) and (2p, s, Ar-$CH_2$—N) respectively.

UV (0.5NHCl in 50/50 methanol/water)
λmax = 273 nm
ε = $1.93 \times 10^3$
λmin = 234 nm

| Elemental analysis | C | H | N | F |
|---|---|---|---|---|
| Found (%) | 46.5 | 4.0 | 13.6 | 37.1 |
| Calculated (%) (as $C_8H_8F_4$) | 46.1 | 3.9 | 13.5 | 36.5 |

## Example 2

Tetrafluoroterephthalonitrile (5 g), methanol (70 ml), water (10 ml), 98% sulphuric acid (3.5 g) and 5% palladium on carbon catalyst (0.25 g) were loaded into the glass liner of a rotating autoclave, purged with nitrogen, then pressurised with hydrogen to 30 atmospheres. The autoclave was rotated for 5 hours, during which time the temperature rose from 15°C to 18°C, and the pressure declined to 28 atmospheres. A slurry of catalyst and solid 2,3,5,6-tetrafluoroxylylene diamine sulphate was filtered. The methanol was removed from the filtrate by reduced pressure distillation, and the aqueous residue, together with additional water, used to completely dissolve the separated solid sulphate product. 5 ml. of this aqueous solution was added to 25 ml 10N sodium hydroxide solution, and extracted with four 10 ml aliquots of diethyl ether. GLC analysis of the combined aliquots showed the yield of 2,3,5,6-tetrafluoroxylylene diamine to be 94.0% with 0.3% 4-cyano-2,3,5-tetrafluorobenzylamine, and no 4-aminomethyl-2,3,5-tetrafluoro-benzylalcohol or 2,3,5,6-tetrafluorobenzylamine.

## Examples 3 to 6

Further tetrafluoroterephthalonitrile reductions were carried out according to Example 2, but with the autoclave charges and reaction conditions summarised in Table I. It is to be noted that the higher pressure used favoured higher diamine yields.

TABLE I

Hydrogenation of tetrafluoroterephthalonitrile in rotating autoclave

| Example No. | Reactor Charge | | | | | Conditions | | | Yields | | | |
|---|---|---|---|---|---|---|---|---|---|---|---|---|
| | Nitrile g. | Methanol ml. | Water ml. | $H_2SO_4$ g. | Cat. g. | Temp. °C | Press. at. | Time hrs. | DA | CA | AA | BA |
| 3 | 15 | 70 | 20 | 9.0 | 0.2 | 12—15 | 30—20 | 6 | 87.4 | 0 | 2.0 | 0.9 |
| 4 | 10 | 70 | 10 | 6.0 | 0.125 | 12 | 30—25 | 6 | 86.3 | 0.8 | | 1.8 |
| 5 | 5 | 70 | 10 | 3.5 | 0.125 | 14 | 7—5 | 6 | 81.5 | 1.1 | 4.5 | 3.8 |
| 6 | 5 | 70 | 10 | 3.5 | 0.25 | 12—19 | 7—4 | 7 | 75.2 | 0.3 | 12.6 | 2.6 |
| 7 | 10 | 40 | 40 | 6 | 0.125 | 10 | 30—20 | 6 | 96.0* | 0 | 0 | 0 |

DA = 2,3,5,6-tetrafluoroxylylene diamine
CA = 4-cyano-2,3,5,6-tetrafluorobenzylamine
AA = 4-aminomethyl-2,3,5,6-tetrafluorobenzyl alcohol
BA = 2,3,5,6-tetrafluorobenzylamine
* conversion 61%

Example 8

Tetrafluoroterephthalonitrile (30 g), methanol (420 ml), water (90 ml), 98% sulphuric acid (21 g) and 5% palladium on carbon catalyst (1.5 g) were loaded to a 1 litre 316 stainless steel autoclave, fitted with a glandless agitator and gas recirculation facility. The autoclave was purged with nitrogen, and the contents maintained under 30 atmospheres of hydrogen pressure while agitation was continued for 6 hours. During this time the temperature rose from 20°C to 28°C. The product slurry was treated and analysed as in Example 2. The yield of 2,3,5,6-tetrafluoroxylylene diamine was 91%, with 0.3% of 2,3,5,6-tetrafluorobenzylamine.

Examples 9 to 13

Further tetrafluoroterephthalonitrile reductions were carried out according to Example 8, but with the autoclave charges and reaction conditions summarised in Table II. Again, the higher pressures gave superior diamine yields.

TABLE II

Hydrogenation of tetrafluoroterephthalonitrile in stirred autoclave

| Example No. | Reactor Charge | | | | | Conditions | | | Yields | | | |
|---|---|---|---|---|---|---|---|---|---|---|---|---|
| | Nitrile g. | Methanol ml. | Water ml. | $H_2SO_4$ g. | Cat. g. | Temp. °C | Press. at. | Time hrs. | DA | CA | AA | BA |
| 9 | 30 | 420 | 90 | 21 | 1.5 | 5—15 | 32 | 6 | 88 | 0 | 0 | 0.04 |
| 10 | 10 | 280 | 20 | 7 | 0.25 | 17—15 | 7 | 6 | 86 | 0 | 1.0 | 4 |
| 11 | 20 | 420 | 60 | 14 | 1.0 | 20—18 | 30 | 6 | 83.3 | 0.1 | 1.9 | 0.4 |
| 12 | 10 | 280 | 20 | 7 | 0.25 | 15—18 | 3.5 | 6 | 75 | 0.3 | 1.6 | 0.8 |
| 13 | 20 | 280 | 20 | 11 | 0.5 | 20—15 | 28 | 6 | 65 | 3.8 | 3.0 | 8.4 |

Example 14

Tetrafluoroterephthalonitrile (5 g), methanol (70 ml), 98% sulphuric acid (3.5 g) and 5% palladium on carbon catalyst (0.125 g) were vigorously agitated under hydrogen at atmospheric pressure for 4.25 hours at 20°C. The resulting slurry was filtered, water added to the filtrate and the methanol removed by reduced pressure distillation. The sulphates in the residue from the hydrogenation were dissolved in the resulting aqueous solution. The products of the hydrogenation were assessed by the procedure described in Example 2. Yields were 27.4% 2,3,5,6-tetrafluoroxylylenediamine, 7.6% 4-cyano-2,3,5,6-tetrafluorobenzylamine and 9.2% 2,3,5,6-tetrafluorobenzylamine.

Examples 15 to 25

Further tetrafluoroterephthalonitrile hydrogenations were carried out according to Example 14, but with varying catalyst and acid additions, and in some Examples, water addition. Results are described in Table III. Water is seen to enhance markedly the diamine yield.

TABLE III

Hydrogenation of tetrafluoroterephthalonitrile at atmospheric pressure

| Example No. | Reactor Charge | | | | Conditions | | | Yields | | | |
|---|---|---|---|---|---|---|---|---|---|---|---|
| | Nitrile g. | Methanol ml. | Water ml. | $H_2SO_4$ g. | Cat. g. | Temp. °C | Time hrs. | DA | CA | AA | BA |
| 15 | 5 | 70 | 0 | 3.5 | 0.06 | 20 | 7.0 | 19.8 | 14.5 | 0 | 11.9 |
| 16 | 5 | 70 | 0 | 3.5 | 0.5 | 20 | 16.5 | 18 | 0 | 2.6 | 2.0 |
| 17 | 5 | 70 | 0 | 3.5 | 0.06 | 55 | 6 | 15.5 | 2.4 | 0 | |
| 18 | 5 | 70 | 5 | 3.0 | 0.25 | 20 | 3 | 65 | 4.8 | 1.1 | 13.7 |
| 19 | 5 | 70 | 5 | 3.5 | 0.125 | 17 | | 53 | 3 | 0.6 | 17.0 |
| 20 | 5 | 70 | 10 | 2.75 | 0.25 | 20 | 4.5 | 49 | 6.5 | 0.7 | 14.1 |
| 21 | 5 | 70 | 10 | 3.0 | 0.25 | 20 | 2.25 | 60 | 1.0 | 11.7 | 3.6 |
| 22 | 5 | 70 | 10 | 3.0 | 0.125 | 19 | 4 | 49 | 10.5 | 0 | 14.4 |
| 23 | 5 | 70 | 10 | 3.5 | 0.125 | 20 | 4 | 50 | 1.4 | 4.0 | 19.6 |
| 24 | 5 | 70 | 10 | 4.0 | 0.125 | 20 | 4 | 50 | 0.4 | 10.0 | 14.4 |
| 25 | 5 | 70 | 10 | 5.0 | 0.125 | 20 | 4 | 38.3 | 0 | 6.7 | 25.2 |

Examples 26, 27 and 28

Tetrafluoroterephthalonitrile (2.5 g), methanol (70 ml) and 5% palladium on carbon catalyst (0.125 g) were loaded to the glass liner of a rotating autoclave, together with 98% sulphuric acid and water in the amounts indicated in Table IV. After purging with nitrogen, the autoclave was pressurised to 10 atmospheres with hydrogen, rotated and heated for the time shown in Table IV. The product suspension was filtered, and the residue and filtrate quantitatively analysed for 2,3,5,6-tetrafluoroxylylene diamine by high pressure liquid chromatography.

If Examples 26 and 27 were carried out at 75°C, it could be expected that the UV spectrum of the solution phase would indicate the presence of 4-cyano-2,3,5,6-tetrafluorobenzylamine (λmax = 234 nm in 0.5N HCl).

Examples 26 to 28 illustrate the beneficial effect of water and the better yield obtained when using a higher acid:nitrile molar ratio.

TABLE IV

| Example No. | $H_2SO_4$:nitrile (molar ratio) | Water ml. | Temp. °C. | Time hrs. | % Yield diamine | | |
|---|---|---|---|---|---|---|---|
| | | | | | Solution | Solid | Total |
| 26 | 1.6:1 | 0 | 90 | 3 | 48 | 35.3 | 83.3 |
| 27 | 1.2:1 | 0 | 92 | 6 | 10 | 46 | 56 |
| 28 | 1.2:1 | 2 | 75 | 6 | 8 | 64.1 | 72.1 |

Example 29

Tetrafluoroterephthalonitrile (10 g), 74 OP ethanol (70 ml), water (5 ml), 98% sulphuric acid (5.4 g) and 5% palladium on charcoal catalyst (0.5 g) were charged to the glass liner of a rotating autoclave and pressurised to 15 atmospheres with hydrogen. The autoclave was rotated at 60°C for 6 hours. The resulting slurry was filtered; high pressure liquid chromatography showed the 2,3,5,6-tetrafluoroxylylene diamine yield to be 72.2% and all in the solid residue.

Example 30

Tetrafluoroterephthalonitrile (2.5 g) 5% palladium on charcoal catalyst (0.125 g), sulphuric acid (6.4 g) and methanol (70 ml) were charged to a glass lined rotary autoclave. The autoclave was pressurised to 50 atmospheres with hydrogen, and rotated for 4 hours at 110°C. The resulting slurry was cooled, filtered and the solid phase recrystallised from a mixture of water and methanol. Elemental analysis, fluorine nmr, proton nmr, infra red and mass spectra (the latter after the sample was heated with sodium bicarbonate), were consistent with the recrystallised material being tetrafluoroxylylene diamine sulphate.

The material gave a single peak on a high pressure liquid chromatogram, when using a mixed ion pair/electrolyte elution system.

Example 31

This example is included for comparative purposes only.

Tetrafluoroterephthalonitrile (2.5 g), nickel catalyst (Harshaw 5132P) (0.8 g), methanol (70 ml) and ammonia (12 g) were charged to a glass lined rotating autoclave. After pressurising to 30 atmospheres with hydrogen, the autoclave was rotated at 110°C for 3 hours. After cooling, filtering off the catalyst, and removing residual ammonia and methanol by distillation, a brown solid remained. Infra red spectroscopy indicated this to be a high molecular weight material, with some loss of ring fluorine, and the presence of an amine hydrohalide.

**Claims**

1. A process for hydrogenating a perhalogenated terephthalonitrile of the formula (I):

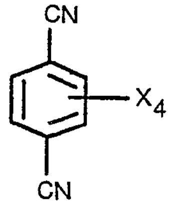

(I)

in which each X is independently fluoro or chloro, to its corresponding amine of formula (II):

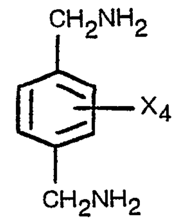

(II)

which comprises reacting under acid conditions the terephthalonitrile with hydrogen in the presence of (i) a hydrogenation catalyst which includes as one component a metal selected from rhodium, palladium, ruthenium, nickel, cobalt and platinum, and (ii) a solvent which is inert to the reaction ingredients and which does not poison the catalyst.

2. A process according to claim 1 for hydrogenating tetrafluoroterephthalonitrile to form 2,3,5,6-tetrafluoroxylylene diamine.

3. A process for hydrogenating a perhalogenated terephthalonitrile of the formula (I):

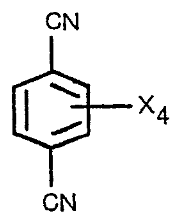

(I)

in which each X is independently fluoro or chloro, to its corresponding amine of formula (II):

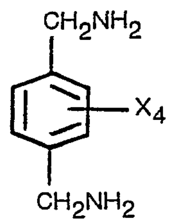

(II)

which comprises reacting the terephthalonitrile with hydrogen under a pressure of 1 to 100 atmospheres and at a temperature of from 0 to 200°C in the presence of (i) a hydrogenation catalyst containing 0.1 to 70% by weight of a metal selected from rhodium, palladium, ruthenium, nickel, cobalt and platinum (ii) an inorganic acid in an amount at least chemically equivalent to the amine formed and (iii) a solvent which is inert to the reaction ingredients and which does not poison the catalyst; the concentration of nitrile in the total reaction mixture being from 3 to 25% by weight.

4. A process according to claim 3 in which water is present in such amount that the proportion of water to solvent is from 1:50 to 1:1 parts by weight.

5. A process according to claim 3 or 4 in which the catalyst contains from 1 to 20% by weight of a metal selected from rhodium, palladium, ruthenium and platinum.

6. A process according to any one of claims 3 to 5 in which the solvent is an alcohol.

7. A process according to any one of claims 3 to 6 in which the pressure is from 1 to 30 atmospheres.

8. A process according to any one of claims 3 to 7 in which the temperature is from 10 to 120°C.

9. A process for hydrogenating tetrafluoroterephthalonitrile to form 2,3,5,6-tetrafluoroxylylene diamine, which comprises reacting the nitrile with hydrogen under a pressure of from 1 to 30 atmospheres and at a temperature of from 10 to 120°C in the presence of (i) 0.5 to 5.0% by weight on the nitrile of a hydrogenation catalyst, which contains from 1 to 20% by weight of a metal selected from rhodium, palladium, ruthenium and platinum, (ii) sulphuric acid in an amount at least chemically equivalent to the amine formed and up to five times the chemically equivalent amount, (iii) an alcohol and (iv) water; the proportion of water to alcohol being from 1:50 to 1:1 parts by weight and the concentration of nitrile in the total reaction mixture being from 3 to 15% by weight.

10. 2,3,5,6-Tetrafluoroxylylene diamine and salts thereof.

**Patentansprüche**

1. Verfahren zur Hydrierung eines perhalogenierten Terephthalonitrils der Formel (I)

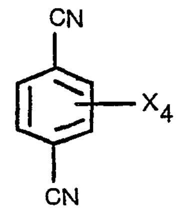

(I)

worin X jeweils unabhängig für Fluoro oder Chloro steht, zum entsprechenden Amin der Formel (II)

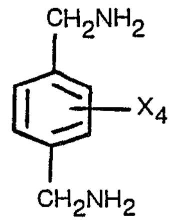

(II)

bei welchem das Terephthalonitril in Gegenwart von (i) einem Hydrierungskatalysator, der als eine Komponente ein aus Rhodium, Palladium, Ruthenium, Nickel, Kobalt und Platin ausgewähltes Metall enthält, und (ii) einem Lösungsmittel, das gegenüber den Reaktionsteilnehmern inert ist und den Katalysator nicht vergiftet, unter sauren Bedingungen mit Wasserstoff umgesetzt wird.

2. Verfahren nach Anspruch 1 zur Hydrierung von Tetrafluoroterephthalonitril zwecks Bildung von 2,3,5,6-Tetrafluoroxylylendiamin.

3. Verfahren zur Hydrierung eines perhalogenierten Terephthalonitrils der Formel (I)

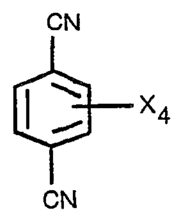

(I)

worin X jeweils unabhängig für Fluoro oder Chloro steht, zum entsprechenden Amin der Formel (II)

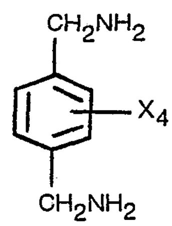

(II)

bei welchem das Terephthalonitril unter einem Druck von 1 bis 100 at und bei einer Temperatur von 0 bis 200°C in Gegenwart von (i) einem Hydrierungskatalysator, der 0,1 bis 70 Gew.-% eines aus Rhodium, Palladium, Ruthenium, Nickel, Kobalt und Platin ausgewählten Metalls enthält, (ii) einer anorganischen Säure in einer Menge, die zumindest chemisch dem gebildeten Amin äquivalent ist, und (iii) einem Lösungsmittel, das gegenüber den Reaktionsteilnehmern inert ist und den Katalysator nicht vergiftet, mit Wasserstoff umgesetzt wird, wobei die Konzentration des Nitrils im gesamten Reaktionsgemisch 3 bis 25 Gew.-% beträgt.

4. Verfahren nach Anspruch 3, bei welchem Wasser in einer solchen Menge vorliegt, daß das Gewichtsverhältnis von Wasser zu Lösungsmittel 1:50 bis 1:1 beträgt.

5. Verfahren nach Anspruch 3 oder 4, bei welchem der Katalysator 1 bis 20 Gew.-% eines aus Rhodium, Palladium, Ruthenium und Platin ausgewählten Metalls enthält.

6. Verfahren nach einem der Ansprüche 3 bis 5, bei welchem das Lösungsmittel ein Alkohol ist.

7. Verfahren nach einem der Ansprüche 3 bis 6, bei welchem der Druck 1 bis 30 at beträgt.

8. Verfahren nach einem der Ansprüche 3 bis 7, bei welchem die Temperatur 10 bis 120°C beträgt.

9. Verfahren zur Hydrierung von Tetrafluoroterephthalonitril zwecks Bildung von 2,3,5,6-Tetrafluoroxylylendiamin, bei welchem das Nitril unter einem Druck von 1 bis 30 at und bei einer Temperatur von 10 bis 120°C in Gegenwart von (i) 0,5 bis 5,0 Gew.-%, bezogen auf das Nitril, eines Hydrierungskatalysators, der 1

bis 20 Gew.-% eines aus Rhodium, Palladium, Ruthenium und Platin ausgewählten Metalls enthält, (ii) Schwefelsäure in einer Menge, die zumindest chemisch dem gebildeten Amin äquivalent ist und bis zum 5fachen der chemisch äquivalenten Menge reichen kann, (iii) einem Alkohol und (iv) Wasser mit Wasserstoff umgesetzt wird, wobei das Gewichtsverhältnis von Wasser zum Alkohol 1:50 bis 1:1 beträgt und die Konzentration des Nitrils im gesamten Reaktionsgemisch 3 bis 15 Gew.-% ausmacht.

10. 2,3,5,6-Tetrafluoroxylylendiamin sowie die Salze davon.

**Revendications**

1. Procédé d'hydrogénation d'un téréphtalonitrile perhalogéné de formule (I):

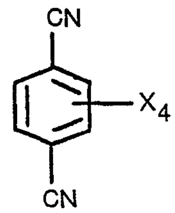

(I)

dans laquelle chaque X représente, indépendamment, un radical fluoro ou chloro, en son amine correspondante de formule (II):

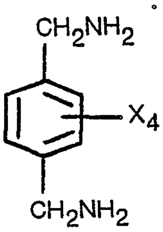

(II)

qui consiste à faire réagir dans des conditions acides le téréphtalonitrile avec de l'hydrogène en présence (i) d'un catalyseur d'hydrogénation qui renferme comme l'un de ses composants un métal choisi entre le rhodium, le palladium, le ruthénium, le nickel, le cobalt et le platine et (ii) un solvant qui est inerte envers les ingrédients réactionnels et qui n'empoisonne pas le catalyseur.

2. Procédé suivant la revendication 1, pour l'hydrogénation de tétrafluorotéréphtalonitrile pour former la 2,3,5,6-tétrafluoroxylylène-diamine.

3. Procédé d'hydrogénation d'un téréphtalonitrile perhalogéné de formule (I):

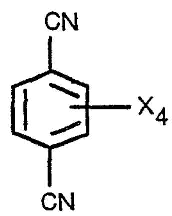

(I)

dans laquelle chaque X représente, indépendamment, un radical fluoro ou chloro, en son amine correspondante de formule (II):

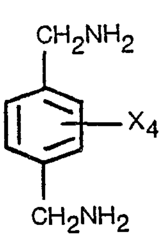

(II)

qui consiste à faire réagir le téréphtalonitrile avec de l'hydogène sous pression de 1 à 100 atmosphères et à une température de 0 à 200°C en présence (i) d'un catalyseur d'hydrogénation contenant 0,1 à 70% en poids d'un métal choisi entre le rhodium, le palladium, le ruthénium, le nickel, le cobalt et le platine, (ii) un acid inorganique en une quantité équivalent au moins chimiquement à l'amine formée et (iii) un solvant qui est inerte envers les ingrédients réactionnels et qui n'empoisonne pas le catalyseur; la concentration en nitrile dans le mélange réactionnel total étant de 3 à 25% en poids.

4. Procédé suivant la revendication 3, dans lequel l'eau est présente en quantité telle que le rapport de l'eau au solvant soit de 1:50 à 1:1 parties en poids.

5. Procédé suivant la revendication 3 ou 4, dans lequel le catalyseur contient 1 à 20% en poids d'un métal choisi entre le rhodium, le palladium, le ruthénium et le platine.

6. Procédé suivant l'une quelconque des revendications 3 à 5, dans lequel le solvant est un alcool.

7. Procédé suivant l'une quelconque des revendications 3 à 6, dans lequel la pression va de 1 à 30 atmosphères.

8. Procédé suivant l'une quelconque des revendications 3 à 7, dans lequel la température va de 10 à 120°C.

9. Procédé d'hydrogénation de tétrafluorotéréphtalonitrile pour former la 2,3,5,6-tétrafluoroxylylène-diamine, qui consiste à faire réagir le nitrile avec de l'hydrogène sous une pression de 1 à 30 atmosphères et à une température de 10 à 120°C en présence (i) de 0,5 à 5,0% en poids, par rapport au nitrile, d'un catalyseur d'hydrogénation qui contient 1 à 20% en poids d'un métal choisi entre le rhodium, le palladium, le ruthénium et le platine, (ii) de l'acide sulfurique en une quantité qui équivaut au moins chimiquement à l'amine formée et qui va jusqu'à 5 fois la quantité chimiquement équivalente, (iii) d'un alcool et (iv) d'eau; le rapport de l'eau à l'alcool en parties en poids allant de 1:50 à 1:1 et la concentration du nitrile dans le mélange réactionnel total allant de 3 à 15% en poids.

10. La 2,3,5,6-tétrafluoroxylylène-diamine et ses sels.